Europäisches Patentamt
European Patent Office
Office européen des brevets

(19)

(11) Publication number: **0 071 374**
**A1**

(12) # EUROPEAN PATENT APPLICATION

(21) Application number: 82303761.9

(22) Date of filing: 16.07.82

(51) Int. Cl.[3]: C 07 C 127/15
A 61 K 31/17

(30) Priority: 21.07.81 JP 114213/81

(43) Date of publication of application:
09.02.83 Bulletin 83/6

(84) Designated Contracting States:
BE CH DE FR GB IT LI SE

(71) Applicant: KUREHA KAGAKU KOGYO KABUSHIKI KAISHA
9-11 Horidome-cho 1-chome Nihonbashi Chuo-ku
Tokyo(JP)

(72) Inventor: Yoshikumi, Chikao
2-19-46 Higashi
Kunitachi-shi Tokyo(JP)

(72) Inventor: Furusyo, Takao
1-6-13 Asahi-machi
Machida-shi Tokyo(JP)

(72) Inventor: Fujii, Takayoshi
5-11-26 Towa
Adachi-ku Tokyo(JP)

(72) Inventor: Saito, Kenichi
3-26-2-204 Hyakunin-cho
Shinjuku-ku Tokyo(JP)

(72) Inventor: Fujii, Masahiko
Mezon Shirayuri 1-6-11 Nakaizumi
Komae-shi Tokyo(JP)

(72) Inventor: Niimura, Kouichi
31-201 Shinsayama-Haitsu 63 Aoyagi
Sayama-shi Saitama-ken(JP)

(74) Representative: Myerscough, Philip Boyd et al,
J.A.Kemp & Co. 14, South Square Gray's Inn
London, WC1R 5EU(GB)

(54) **Nitrosourea derivatives having anti-tumour and anti-bacterial activity.**

(57) Nitrosourea derivatives represented by the formula (I):

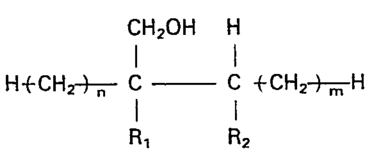

(I)

wherein $R_1$ is hydroxy and $R_2$ is

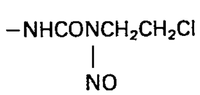

or $R_1$ is

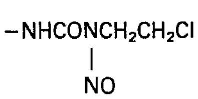

and $R_2$ is hydroxy, and m and n each denote O or an integer of from 1 to 4, with the proviso that $R_1$ is not

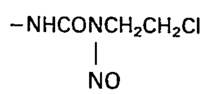

and $R_2$ is not hydroxy when m and n are both O, are useful as anti-tumour and anti-bacterial agents.

EP 0 071 374 A1

DESCRIPTION

"NITROSOUREA DERIVATIVES HAVING ANTI-TUMOR AND ANTI-BACTERIAL ACTIVITY"

The present invention relates to nitrosourea derivatives, their preparation and pharmaceutical compositions containing them.

Many halogenated alkylnitrosoureas are known. Amongst those which are well known are 1,3-bis-(2-chloroethyl)-1-nitrosourea (hereinafter referred to as BCNU), 1-(2-chloroethyl)-3-cyclohexyl-1-nitrosourea (hereinafter referred to as CCNU) and 1-(2-chloroethyl)-3-(4-methylcyclohexyl)-1-nitrosourea (hereinafter referred to as Me-CCNU).

However, since the above-mentioned compounds are oil-soluble, they are administered after dissolution in ethanol and accordingly, the administration of every one of them presses a burden on the patients to whom the compound is administered as well as injures the bone marrow, lymphatic gland, kidney, lung, liver and gastrointestinal system.

The present invention is concerned with new derivatives of nitrosourea which are soluble in water and retain the useful properties of the known derivatives of nitrosourea while not showing the undesirable side effects mentioned above.

Accordingly, the present invention provides a derivative of nitrosourea represented by the formula (I) :

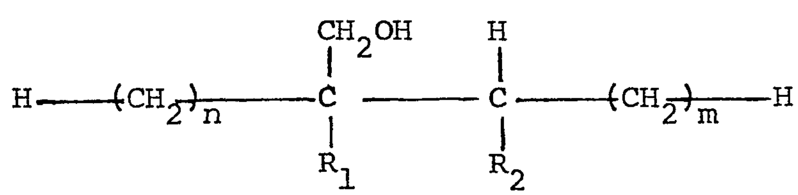

(I)

wherein $R_1$ is hydroxy and $R_2$ is 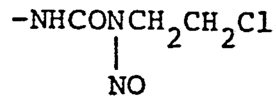 or $R_1$ is 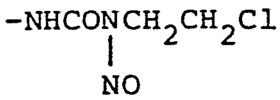 and $R_2$ is hydroxy, and m and n each denote 0 or an integer of 1 to 4 providing that $R_1$ is not 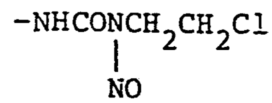 and $R_2$ is not hydroxy when m and n are both 0.

In a second aspect of the present invention, there is provided a process for preparing a derivative of nitrosourea represented by the formula (I):

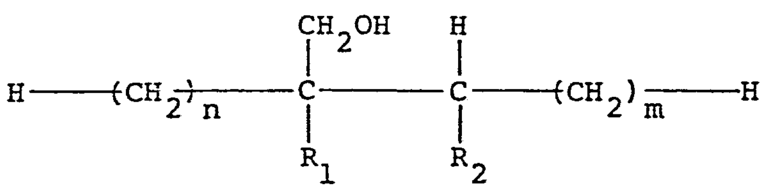 (I)

wherein $R_1$, $R_2$, m and n are defined in the above, comprising reacting an amino-alcohol represented by the formula (II):

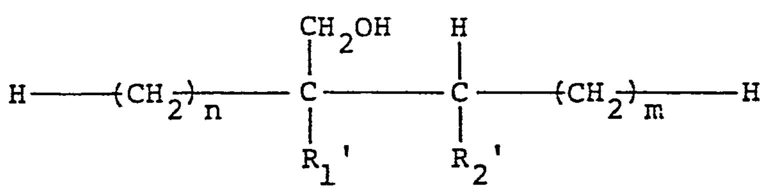 (II)

wherein $R_1'$ is hydroxy and $R_2'$ is amino or $R_1'$ is amino and $R_2'$ is hydroxy, and m and n are each 0 or an integer of 1 to 4 providing that $R_1'$ is not amino and $R_2'$ is not hydroxy when m and n are 0, with a chloroethyl isocyanate to prepare an intermediate compound represented by the formula (IV):

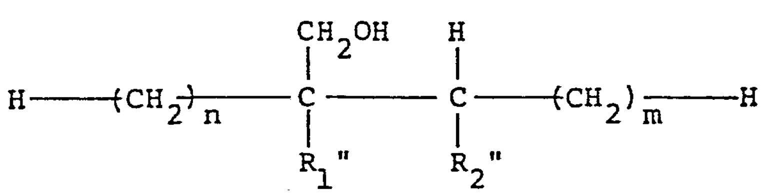 (IV)

wherein $R_1$" is hydroxy and $R_2$" is $-NHCONHCH_2CH_2Cl$ or $R_1$" is $-NHCONHCH_2CH_2Cl$ and $R_2$" is hydroxy, and m and n are each 0 or an integer of  to 4 providing that $R_1$" is not $-NHCONHCH_2CH_2Cl$ and $R_2$" is not hydroxy when m and n are 0, and reacting the intermediate compound thus prepared with a nitrous acid.

In a third aspect of the present invention, there is provided a process for preparing a derivative of nitrosourea represented by the formula (I):

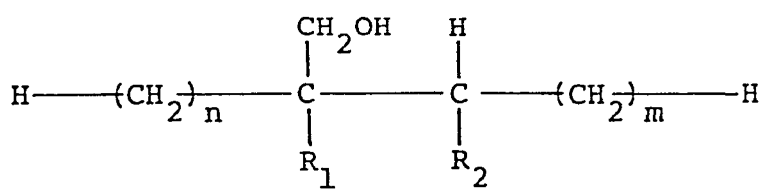

(I)

wherein $R_1$, $R_2$, m and n are defined in the above, comprising reacting an amino-alcohol represented by the formula (II):

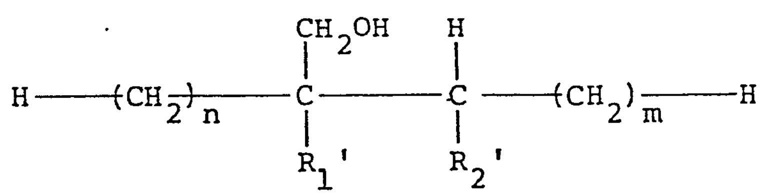

(II)

wherein $R_1'$, $R_2'$, m and n are defined in the above, with N-(2-chloroethyl)-N-nitrosocarbamylazide or p-nitrophenyl N-(2-chloroethyl)-N-nitrosocarbamate.

In the fourth aspect of the present invention, there is provided a pharmaceutical composition containing a derivative of nitrosourea represented by the formula (I) :

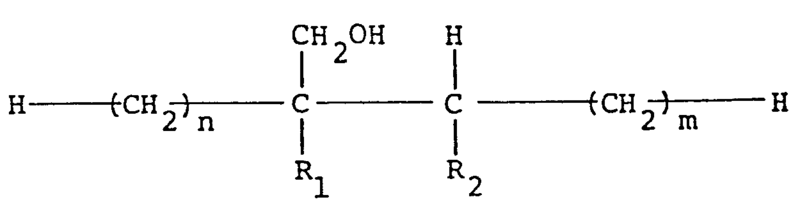

(I)

wherein $R_1$, $R_2$, m and n are defined in the above, and a pharmaceutically acceptable carrier.

The novel compounds according to the present invention are derivatives of nitrosourea (hereinafter referred to as the present compounds) represented by the formula (I):

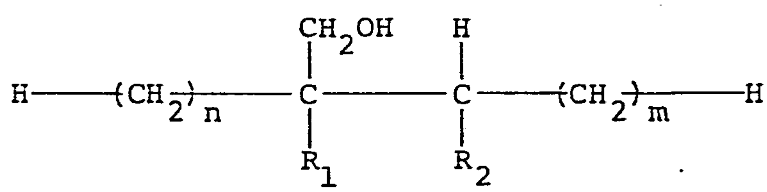 (I)

wherein $R_1$ is hydroxy and $R_2$ is 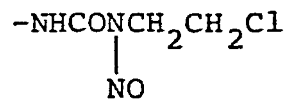 or $R_1$ is 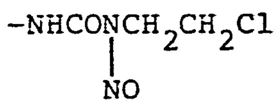 and $R_2$ is hydroxy, and m and n denote respectively an integer of 0 to 4 providing that $R_1$ is not 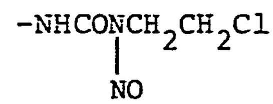 and $R_2$ is not hydroxy when m and n are 0.

The present compounds represented by the formula (I) include the following compounds:

2-Methyl-2-[3-(2-chloroethyl)-3-nitrosoureido]-1,3-propanediol,

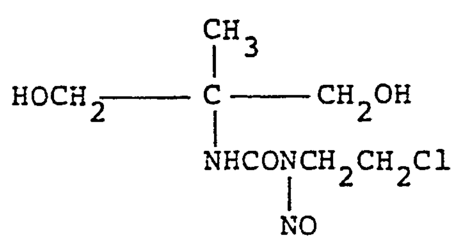

2-Ethyl-2-[3-(2-chloroethyl)-3-nitrosoureido]-1,3-propanediol,

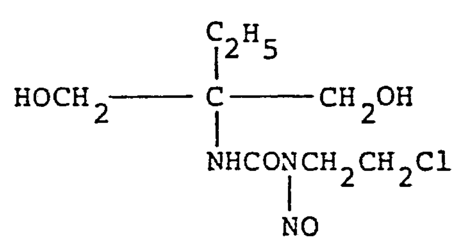

2-Propyl-2-[3-(2-chloroethyl)-3-nitrosoureido]-1,3-propanediol,

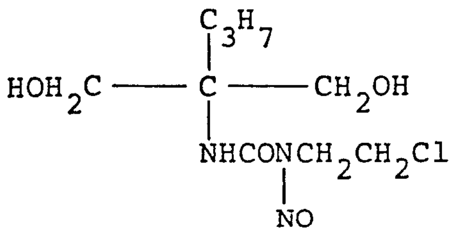

2-Butyl-2-[3-(2-chloroethyl)-3-nitrosoureido]-1,3-propanediol,

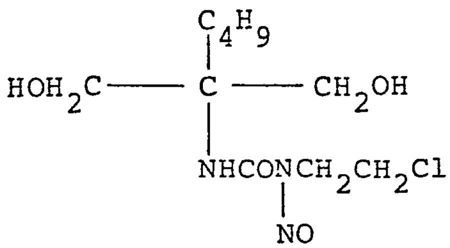

3-[3-(2-Chloroethyl)-3-nitrosoureido]-1,2-propanediol,

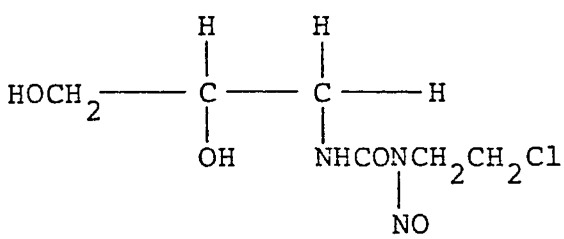

3-[3-(2-Chloroethyl)-3-nitrosoureido]-1,2-butanediol,

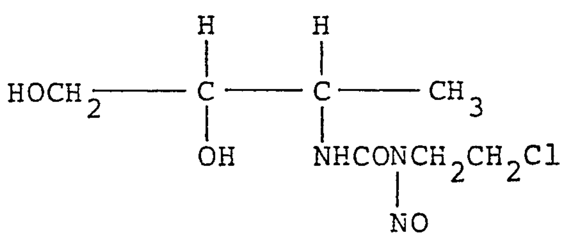

3-[3-(2-Chloroethyl)-3-nitrosoureido]-1,2-pentanediol,

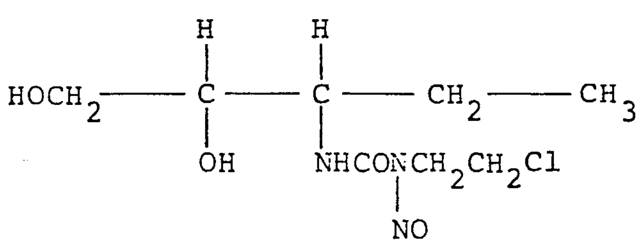

3-[3-(2-Chloroethyl)-3-nitrosoureido]-1,2-hexanediol,

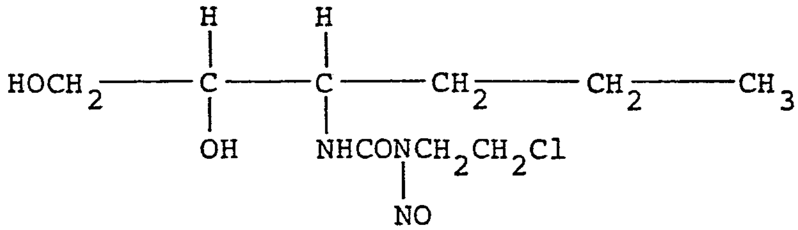

3-[3-(2-Chloroethyl)-3-nitrosoureido]-1,2-heptanediol.

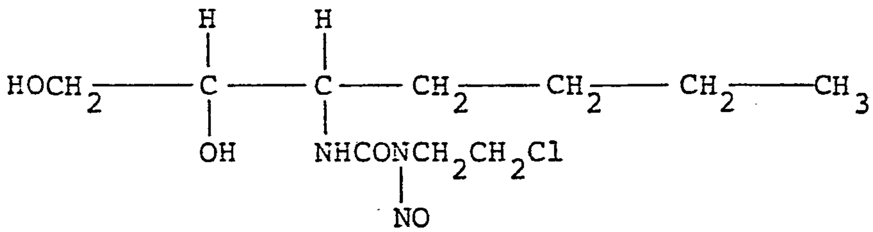

The present compounds have excellent pharmacological activities such as anti-tumour and anti-bacterial activities and are pharmacologically safe. Accordingly, the present compounds are important compounds as an active ingredient of a pharmaceutical composition in treating various tumours and infections.

The present compounds are prepared as follows: An amino-alcohol represented by the formula (II)

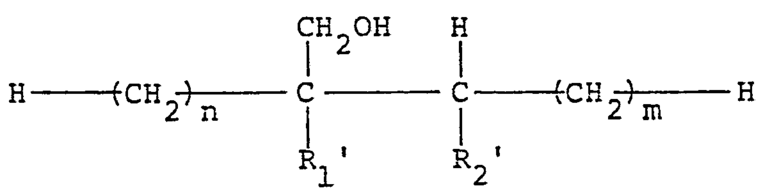

(II)

wherein $R_1$' is a hydroxy group and $R_2$' is an amino group or $R_1$' is an amino group and $R_2$' is a hydroxy group, and m and n are respectively an integer of 0 to 4 providing that $R_1$' is not an amino group and $R_2$' is not a hydroxy group when m and n are 0, is brought into reaction with chloroethyl isocyanate represented by the formula (III)

BAD ORIGINAL

$Cl-CH_2CH_2-N=C=O$ (III)

to be converted to an intermediate compound represented by the formula (IV)

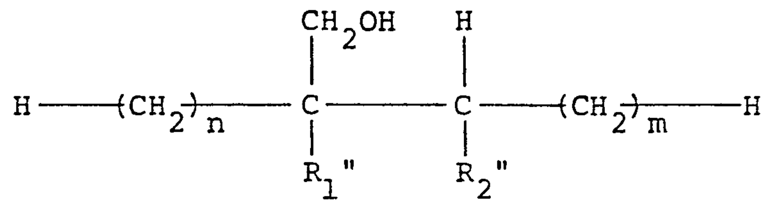 (IV)

wherein $R_1"$ is hydroxy and $R_2"$ is 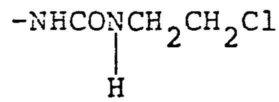 or $R_1"$ is 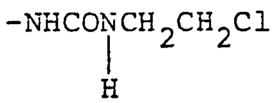 and $R_2"$ is hydroxy, and m and n are respectively an integer of 0 to 4 providing that $R_1"$ is not 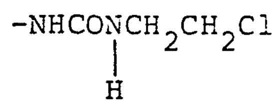 and $R_2"$ is not hydroxy when m and n are 0. This reaction is carried out in a polar solvent such as methanol or ethanol, or in dioxane and further in a mixed solvent of water and one of these solvents at a temperature of -30 to 50°C for 1 to 48 hours. Generally, chloroethyl isocyanate is added to a solution of the amino-alcohol while stirring at a predetermined temperature in an amount of from equimolar to an excess of 2 molar, preferably a little more than equimolar amount. The intermediate compound (IV) is available by evaporating the solvent from the reaction mixture under a reduced pressure. And then, the intermediate compound (IV) is brought into reaction with nitrous acid which is formed from a metal nitrite such as sodium nitrite and an organic or inorganic acid and represented by the formula (V)

$HNO_2$ (V)

BAD ORIGINAL

to obtain the present compound. The second reaction is carried out in a polar solvent containing an organic substance. The most convenient solvent is an aqueous acidic solution of a lower carboxylic acid, for instance, formic acid and acetic acid, into which a nitrite such as sodium nitrite is added. The reaction is carried out at -30 to 50°C for 1 to 48 hours. The reaction product is easily extracted from the reaction mixture with an organic solvent such as chloroform or ethyl acetate or with a mixed solvent of water and the organic solvent. In addition, the excess amount of metallic ions are removed by using an ionexchange resin. The thus obtained solution is condensed under a reduced pressure to give the isolated product. The product is recrystallized if necessary.

Another method for preparing the present compounds is as follows:
An amino-alcohol represented by the formula (II) is added to a solution of N-(2-chloroethyl)-N-nitrosocarbamylazide represented by the formula (VI)

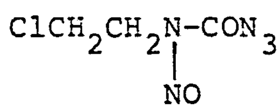

(VI)

or p-nitrophenyl N-(2-chloroethyl)-N-nitrosocarbamate represented by the formula (VII)

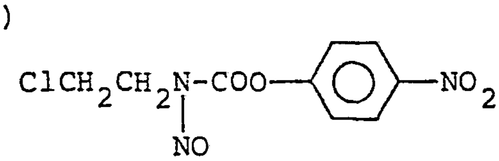

(VII)

in an alcohol such as isopropyl alcohol or tetrahydrofuran at a temperature of -30 to 50°C to be reacted for 1 to 48 hours.

BAD ORIGINAL

The reaction mixture is subjected to extraction with an organic solvent such as chloroform and ethyl acetate to obtain the present compound, which is recrystallized if necessary.

The present compound shows anti-tumour and anti-bacterial activities and a low acute toxicity, as is shown in Examples. Accordingly, the present compound is a useful anti-tumour agent as itself or an active ingredient of pharmaceutical composition for treating several tumours such as Sarcoma-180, P-388 leukemia, L-1210 leukemia, Ehrlich's cancer and Yoshida sarcoma on animals, and cancers of the ovary, testis, head and neck, urinary bladder, breast, uterine cervix, and the like. And, the present compound has an anti-bacterial activity against gram-negative bacteria such as Escherichia coli, and accordingly, is used as an anti-infective disease agent for treating an infection, particularly as an anti-bacterial agent for treating a bacterial infection.

Furthermore, the present compound may be used as an active ingredient of a pharmaceutical composition for the above-mentioned diseases.

The present compound can be administered orally or parenterally in the various dosage forms as a composition together with a pharmaceutically acceptable carrier and/or an adjuvant. The composition may be in unit dosage form.

The dosage form of the composition may be tablet, sugar-coated tablet, pill, powder, granule, capsule, trouch, solution, suppository and injection. An example of the carrier mentioned above is lactose, saccharose, glucose, sorbitol,

BAD ORIGINAL

mannitol, potato- or corn starch, amiropectin, various derivatives of starch and cellulose such as carboxymethylcellulose and hydroxypropylcellulose, gelatin, magnesium stearate, calcium stearate, titanium dioxide, talc, polyvinyl alcohol, polyethylene glycol wax, gum arabic, vegetable oil such as olive oil, peanut oil, sesame oil, paraffin oil, neutral fat base, ethanol, physiological saline solution, sterilized water, glycerol or the like. In the composition, if necessary, tinctorial agent, seasoning agent, thickener, stabilizer, agent for osmoregulation or buffer, that is, the conventional pharmaceutical adjuvant may be used together.

The content of the present compound in the pharmaceutical composition may be varied, however, it is 0.001% - 85 % by weight, preferably 0.005% - 60% by weight of the composition.

A dose of the pharmaceutical composition of the present invention is 0.05 to 200 mg, preferably 0.1 to 100 mg per day per one kilogram of the body weight. However the dose of pharmaceutical composition may be varied while mainly depending on condition of a disease of a human or animal.

The following Examples illustrate the present invention.

PREPARATION OF THE PRESENT COMPOUND:

EXAMPLE 1:

Preparation of 2-methyl-2-[3-(2-chloroethyl)-3-nitrosoureido]-1,3-propanediol:

Into 30 ml of methanol, 3.15 g of 2-amino-2-methyl-1,3-propanediol was dissolved, and after cooling the solution to 0°C, 3.6 g of 2-chloroethyl isocyanate was slowly dropped into the cooled solution. After leaving the mixture for one night, the solvent was distilled off from the reaction mixture under a reduced pressure to obtain an oily residue, which was dissolved in a mixed solvent of 15 ml of water and 15 ml of acetic acid. Into the solution under cooling to 0°C, 4.12 g of sodium nitrite was added, and the mixture was brought into reaction for one night. Then, the solvent was distilled off from the reaction mixture under a reduced pressure, and the residue was subjected to extraction with 20 ml of water and 40 ml of chloroform followed by two times of extraction with each 40 ml of chloroform. After drying the chloroform extract on magnesium sulfate, the extract was condensed under a reduced pressure. The condensate was subjected to chromatography while using 100 g of silica gel to collect the eluate by a mixed solvent of 95:5(vol) of chloroform and methanol, and the eluate was condensed to be a crude product, which was recrystallized from a mixed solvent of ethyl acetate and n-hexane to obtain 1.0g of 2-methyl-2-[3-(2-chloroethyl)-3-nitrosoureido]-1,3-propanediol.

The characteristics of the present compound thus obtained were as follows:

(1) melting point; 55 to 57°C

(2) elementary analysis;

| | C(%) | H(%) | N(%) |
|---|---|---|---|
| theoretical: | 35.08 | 5.88 | 17.53 |
| Experimental: | 35.30 | 6.02 | 17.20 |

(3) infrared absorption (IR) spectrum; shown in Figure 1.

EXAMPLE 2:

Preparation of 2-ethyl-2-[3-(2-chloroethyl)-3-nitrosoureido]-1,3-propanediol:

In a similar manner to Example 1 except for the use of 2-amino-2-ethyl-1,3-propanediol in place of 2-amino-2-methyl-1,3-propanediol of Example 1, 2-ethyl-2-[3-(2-chloroethyl)-3-nitrosoureido]-1,3-propanediol was obtained.

The characteristics of the present compound thus obtained were as follows:

(1) melting point; 78 to 81°C

(2) elementary analysis;

| | C(%) | H(%) | N(%) |
|---|---|---|---|
| theoretical: | 37.88 | 6.36 | 16.56 |
| experimental: | 37.60 | 6.32 | 16.41 |

(3) infrared absorption (IR) spectrum; shown in Figure 2.

EXAMPLE 3:

Preparation of 3-[3-(2-chloroethyl)-3-nitrosoureido]-1,2-propanediol:

Into a cooled solution of 1.8 g of 3-amino-1,2-propanediol dissolved in 20 ml of methanol at -5°C, 2.3 g of chloroethyl isocyanate is slowly dropped, and the mixture was left for one night to react. Then the solvent was distilled off from the reaction mixture under a reduced pressure, and the

residue was dissolved in a mixed solvent of 15 ml of water and 15 ml of acetic acid. While cooling the solution at -5°C, 4.12 g of sodium nitrite was added to the solution and the mixture was left for one night. Then the solvent was distilled off from the reaction mixture under a reduced pressure, and the residue was subjected to extraction three times with each 50 ml of chloroform after adding 20 ml of water. After drying the chloroform extract on magnesium sulfate, the extract was condensed under a reduced pressure and the condensate was subjected to column chromatography while using 100 g of silica gel to collect the eluate by a mixed solvent of 93:7 (vol) of chloroform and methanol. By condensing the eluate, a crude product was obtained, which was recrystallized from a mixed solvent of ethyl acetate and n-hexane to obtain 1.6 g of 3-[3-(2-chloroethyl)-3-nitrosoureido]-1,2-propanediol.

The characteristics of the present compound thus obtained were as follows:

(1) melting point; 46 to 47°C

(2) elementary analysis;

| | C(%) | H(%) | N(%) |
|---|---|---|---|
| theoretical: | 31.93 | 5.37 | 18.62 |
| experimental: | 32.00 | 5.30 | 18.30 |

(3) infrared absorption (IR) spectrum; shown in Figure 3.

EXAMPLE 4:

Preparation of 3-[3-(2-chloroethyl)-3-nitrosoureido]-1,2-butanediol:

Into a cooled solution of 1.9 g of 3-amino-1,2-

butanediol dissolved in 20 ml of methanol at 0°C, 2.4 g of chloroethyl isocyanate was dropped slowly. After leaving the reaction mixture for one night, the solvent was distilled off from the mixture under a reduced pressure, and the residue was dissolved in a mixed solvent of 15 ml of water and 15 ml of acetic acid. While cooling the solution to 0°C, 4.12 g of sodium nitrite was added to the solution and the reaction mixture was left for one night to react. Then the solvent was removed from the solution under a reduced pressure, and the residue was subjected to extraction three times with each 50 ml of chloroform after adding 20 ml of water.

The chloroform extract was dried on magnesium sulfate, condensed under a reduced pressure, and the condensate was subjected to column chromatography while using 100 g of silica gel to collect the eluate by a mixed solvent of 93:7 (vol) of chloroform and methanol. By condesning the eluate, a crude product was obtained, which was recrystallized from a mixed solvent of ethyl acetate and n-hexane to be 1.5 g of 3-[3-(2-chloroethyl)-3-nitrosoureido]-1,2-butanediol.

The characteristics of the present compound thus obtained were as follows:

(1) melting point; 102 to 104°C

(2) elementary analysis;

| | C(%) | H(%) | N(%) |
|---|---|---|---|
| theoretical: | 35.07 | 5.90 | 17.53 |
| experimental: | 35.10 | 5.91 | 17.50 |

(3) infrared absorption (IR) spectrum; shown in Figure 4.

EXAMPLE 5:

Preparation of 2-methyl-2-[3-(2-chloroethyl)-3-nitrosoureido]-1,3-propanediol:

Into a solution of 20 mmol of N-(2-chloroethyl)-N-nitrosocarbamylazide in 10 ml of isopropyl alcohol, a solution of 3.15 g of 2-amino-2-methyl-1,3-propanediol dissolved in 5 ml of isopropyl alcohol was slowly added dropwise at a temperature of 5°C. After stirring the mixture for one night and adjusting the pH of the mixture to be acidic with the addition of 1N $H_2SO_4$, the mixture was extracted with 100 ml of chloroform. After washing the chloroform extract with water and drying on sodium sulfate, the extract was condensed under a reduced pressure and the condensate was recrystallized from a mixed solvent of ethyl acetate and n-hexane to obtain 1.2 g of 2-methyl-2-[3-(2-chloroethyl)-3-nitrosoureido]-1,3-propanediol.

The characteristics of the present compound thus obtained were as follows:

(1) melting point; 55 to 57°C

(2) elementary analysis;

| | C(%) | H(%) | N(%) |
|---|---|---|---|
| theoretical: | 35.08 | 5.88 | 17.53 |
| experimental: | 35.33 | 6.01 | 17.21 |

(3) infrared absorption (IR) spectrum; shown in Figure 5.

EXAMPLE 6:

Preparation of 3-[3-(2-chloroethyl)-3-nitrosoureido]-1,2-propanediol:

A solution of 1.8 g of 3-amino-1,2-propanediol dissolved in 10 ml of tetrahydrofuran was added to a solution of 6.5 g of p-nitrophenyl N-(2-chloroethyl)-N-nitrosocarbamate dissolved in 10 ml of tetrahydrofuran, and the mixture was stirred for one night at 25°C. Then the solvent was distilled off from the mixture under a reduce pressure and the residue was subjected to chromatography by using 100 g of silica gel to collect the eluate with a mixed solvent of 93:7 (vol) of chloroform and methanol. The crude product obtained by condensing the eluate was recrystallized from a mixed solvent of ethyl acetate and n-hexane to obtain 3-[3-(2-chloroethyl)-3-nitrosoureido]-1,2-propanediol.

The characteristics of the present compound thus obtained were as follows:

(1) melting point; 46 to 47°C

(2) elementary analysis;

| | C(%) | H(%) | N(%) |
|---|---|---|---|
| theoretical: | 31.93 | 5.37 | 18.62 |
| experimental: | 32.02 | 5.31 | 18.31 |

(3) infrared absorption (IR) spectrum; shown in Figure 6.

PHARMACOLOGICAL ACTIVITIES OF THE PRESENT COMPOUND:

EXAMPLE 7:

Anti-tumour effect against L-1210 leukemia on mouse:

The successively cultured L-1210 leukemia cells in the ascites of a mouse were transplanted intraperitoneally to a group of the mouse at a rate of $1 \times 10^5$ cells/animal, and

after 24 hours of the transplantation, a solution of each of the present compounds dissolved in an aqueous physiological saline solution was intraperitoneally administered to the group of the mouse once a day for 5 days running. By observing the state of the mice, the average survival days of the administered mice (T) and those of the control group (C) were obtained to calculate the prolongation ratio of the mouse's life (T/C x 100).

The result is shown in Table 1.

As seen from Table 1, the prolongation ratio of the life due to the present compounds was larger than that due to the comparative compound(BCNU).

TABLE 1

| Compound | Dose (mg/kg) | Prolongation ratio (%) |
|---|---|---|
| 2-Methyl-2-[3-(2-chloroethyl)-3-nitrosoureido]-1,3-propanediol of the present compound | 10 | 410 |
| 2-Ethyl-2-[3-(2-chloroethyl)-3-nitrosoureido]-1,3-propanediol of the present compound | 10 | 405 |
| 3-[3-(2-Chloroethyl)-3-nitrosoureido]-1,2-propanediol of the present compound | 10 | 420 |
| 3-[3-(2-Chloroethyl)-3-nitrosoureido]-1,2-butanediol of the present compound | 10 | 430 |
| 1,3-Bis(2-chloroethyl)-1-nitrosourea (BCNU) of the comparative compound | 10 | 204 |

EXAMPLE 8:

Anti-tumour effect against V-7 tumour on a rabbit:

The cancer cells prepared by extirpating the solid tumour transplated and proliferated in the femoral part of a rabbit, cutting finely and having passed through stainless steel mesh of Tyler standard mesh of #200 were transplanted to the thinner muscle of the femoral part of a rabbit by using an injector at a rate of $1.5 \times 10^7$ cells/animal. On the 7th day and 11th day of the transplantation, the present compound was administered to the transplanted rabbit. The prolongation ratio of the rabbit was calculated by the comparison of the survival days of the administered rabbits and the not-administered control rabbits.

The result is shown in Table 2.

TABLE 2

| Compound | Dose (mg/kg) | Prolongation ratio (%) |
|---|---|---|
| 2-Methyl-2-[3-(2-chloroethyl)-3-nitrosoureido]-1,3-propanediol of the present compound | 10 | 205 |
| 2-Ethyl-2-[3-(2-chloroethyl)-3-nitrosoureido]-1,3-propanediol of the present compound | 10 | 230 |
| 3-[3-(2-Chloroethyl)-3-nitrosoureido]-1,2-propanediol of the present compound | 10 | 215 |
| 3-[3-(2-Chloroethyl)-3-nitrosoureido]-1,2-butanediol of the present compound | 10 | 210 |
| 1,3-Bis(2-chloroethyl)-1-nitrosourea (BCNU) of the comparative compound | 10 | 140 |

EXAMPLE 9:

Antibacterial activity:

i) A series of 2 times dilution of aqueous solution of each of the present compounds were prepared, and a plate culture medium was prepared by mixing each series with Heart infusion agar culture medium. A strain of Escherichia coli cultured in trypto-soy bouillon at 37°C for 18 hours was inoculated on the thus prepared culture medium, and after culturing for 18 hours at 37°C, the degree of proliferation of the inoculated bacteria was observed, the inoculum being a plutinum loop-full.

The results are shown in Table 3.

As seen from Table 3, the antibacterial activity of the present compound against Escherichia coli is recognized in vitro.

TABLE 3

| Compound | Diluted concentration | | | |
|---|---|---|---|---|
| | 1 | 1/2 | 1/4 | 1/8 |
| 2-Methyl-2-[3-(2-chloroethyl)-3-nitrosoureido]-1,3-propanediol of the present compound | +++ | ++ | ++ | + |
| 2-Ethyl-2-[3-(2-chloroethyl)-3-nitrosoureido]-1,3-propanediol of the present compound | +++ | ++ | + | + |
| 3-[3-(2-Chloroethyl)-3-nitrosoureido]-1,2-propanediol of the present compound | +++ | ++ | + | + |
| 3-[3-(2-Chloroethyl)-3-nitrosoureido]-1,2-butanediol of the present compound | +++ | ++ | + | + |
| 1,3-Bis(2-chloroethyl)-1-nitrosourea (BCNU) of the comparative compound | + | + | - | - |

Notes: + means the degree of inhibition of the growth of E. Coli; the larger the number of +, the larger the inhibition
- means that no inhibition of the growth of E. coli was observed.

ii) The effect on the treatment of the infection was tested using mouse as the experimental animal.

After 1 to 3 hours of the intraperitoneal inoculation of more than 1 x $10^5$ cells of Escherichia coli to each of the mice, each of the present compounds was administered orally or intraperitoneally to the mouse at a dose rate of 1 to 100 mg/kg and the mouse were observed until 7th day after the administration.

A survival rate higher than 50 % was resulted. This result shows that the present compound is effective in treating the experimental infection.

EXAMPLE 10:

Acute toxicity:

Acute toxicity was examined by administering intraperitoneally an aqueous physiological saline solution in which each of the present compounds is dissolved to ICR-JCL mouse. The state of the toxicosis was observed for 14 days to obtain the mortality as the time passed by, from which median lethal dose ($LD_{50}$) was calculated according to the graphic method of Litchfield-Wilcoxon.

The present compounds showed $LD_{50}$ larger than 60 mg/kg.

EXAMPLE 11:

Side effects:

The side effects of the present compounds were

examined using JCL-ICR mouse as the experimental animal.

Each of the present compounds was orally administered to a group of the mouse at a daily dose rate of 10 mg/kg once a day for 5 days running, the same procedure being carried out on the other group of the mouse by administering BCNU at the same rate.

The results on the nephrotoxicity and the solubility in water were shown in Table 4.

As seen from Table 4, in the mice administered with BCNU, albuminurea was observed severely showing the nephrotoxicity of BCNU, while the degree of albuminurea in the animals administered with the present compound was slight. It is clear as seen in Table 4 that the nephrotoxicity and the solubility in water were improved in the present compound as compared to BCNU.

TABLE 4

| Compound | Nephrotoxicity | Solubility in Water |
|---|---|---|
| 2-Methyl-2-[3-(2-chloroethyl)-3-nitrosoureido]-1,3-propanediol of the present compound | + | good |
| 2-Ethyl-2-[3-(2-chloroethyl)-3-nitrosoureido]-1,3-propanediol of the present compound | + | good |
| 3-[3-(2-Chloroethyl)-3-nitrosoureido]-1,2-propanediol of the present compound | + | good |
| 3-[3-(2-Chloroethyl)-3-nitrosoureido]-1,2-butanediol of the present compound | + | good |
| 1,3-Bis(2-chloroethyl)-1-nitrosourea (BCNU) of the comparative compound | +++ | not soluble |

Notes: + means that albuminurea was observed in one to two animals of the total ten animals.

+++ means that albuminurea was observed in eight to nine animals of the total ten animals.

MANUFACTURE OF THE PHARMACEUTICAL PREPARATIONS:

EXAMPLE 12:

Manufacture of the tablet:

50 Grams of 2-methyl-2-[3-(2-chloroethyl)-3-nitrosoureido]-1,3-propanediol was mixed with 300 g of lactose and 1.5 g of hydroxypropylcellulose, and the mixture was directly pressed to be tablets or after kneading and extruding by a extruding pelletizer to obtain granules, the well dried granules were pressed to be tablets.

EXAMPLE 13:

Manufacture of the injection:

3-[3-(2-chloroethyl)-3-nitrosoureido]-1,2-propanediol was distributed aseptically into glass vials at a rate of 10 mg/vial. After removing the remaining small amount of moisture and bacteria in the vials, the vials were closely sealed. In using, 5 ml of an aqueous 5% by weight solution of glucose for injection was added to the content of each vial to prepare the injection.

CLAIMS

1. A derivative of nitrosourea represented by the formula (I) :

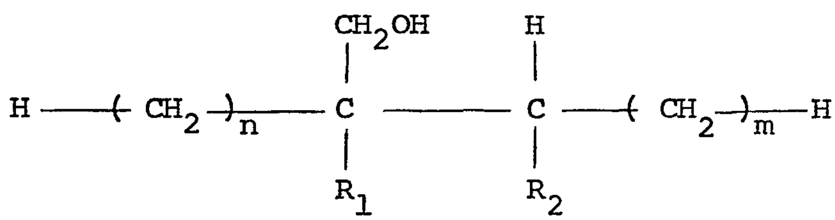 (I)

wherein $R_1$ is hydroxy and $R_2$ is $-NHCON(NO)CH_2CH_2Cl$ or $R_1$ is $-NHCON(NO)CH_2CH_2Cl$ and $R_2$ is hydroxy, and m and n each denote 0 or an integer of from 1 to 4, with the proviso that $R_1$ is not $-NHCON(NO)CH_2CH_2Cl$ and $R_2$ is not hydroxy when m and n are both 0.

2. A derivative according to claim 1, wherein $R_1$ is $-NHCON(NO)CH_2CH_2Cl$, $R_2$ is hydroxy, m is 0 and n is an integer of from 1 to 4.

3. A derivative according to claim 1, wherein $R_1$ is hydroxy, $R_2$ is $-NHCON(NO)CH_2CH_2Cl$, m is 0 or an integer of from 1 to 4 and n is 0.

4. A derivative according to claim 2, which is 2-methyl-2-[3-(2-chloroethyl)-3-nitrosoureido]-1,3-propanediol; 2-ethyl-2-[3-(2-chloroethyl)-3-nitrosoureido]-1,3-propanediol; 2 propyl-2-[3-(2-chloroethyl)-3-nitrosoureido]-1,3-propanediol; or 2-butyl-2-[3-(2-chloroethyl)-3-nitrosoureido]-1,3-propanediol.

5. A derivative according to claim 3, which is 3-[3-(2-chloroethyl)-3-nitrosoureido]-1,2-propanediol; 3-[3-(2-chloroethyl)-3-nitrosoureido]-1,2-butanediol; 3-[3-(2-chloroethyl)-3-nitrosoureido]-1,2-pentanediol; 3-[3-(2-chloroethyl)-3-nitrosoureido]-1,2-hexanediol; or 3-[3-(2-chloroethyl)-3-nitrosoureido]-1,2-heptanediol.

6. A process for preparing a derivative of nitrosourea represented by the formula (I) as defined in claim 1, which process comprises reacting an amino-alcohol represented by the formula (II) :

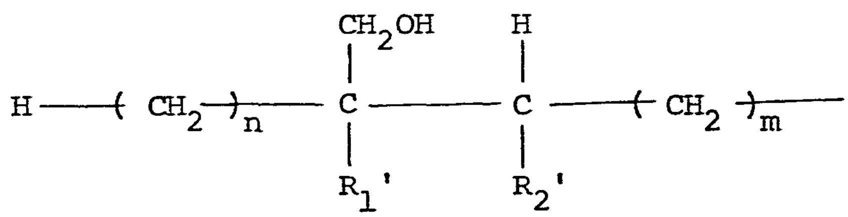

(II)

wherein $R_1'$ is hydroxy and $R_2'$ is amino or $R_1'$ is amino and $R_2'$ is hydroxy, and m and n are each 0 or an integer of from 1 to 4, providing that $R_1'$ is not amino and $R_2'$ is not hydroxy when m and n are both 0, with chloroethyl isocyanate to prepare an intermediate compound represented by the formula (IV) :

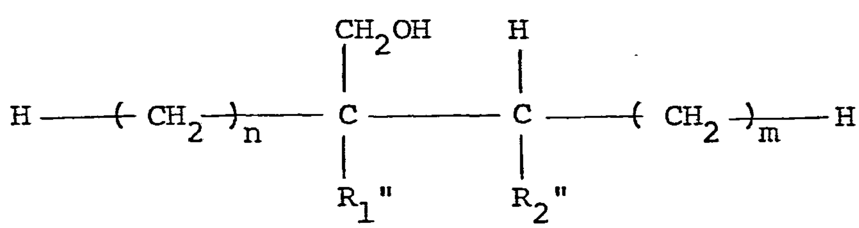

(IV)

wherein $R_1''$ is hydroxy and $R_2''$ is $-NHCONHCH_2CH_2Cl$ or $R_1''$ is $-NHCONHCH_2CH_2Cl$ and $R_2''$ is hydroxy, and m and n are each 0 or an integer of from 1 to 4, providing that $R_1''$ is not $-NHCONHCH_2CH_2Cl$ and $R_2''$ is not hydroxy when m and n are both 0, and reacting the intermediate compound of Formula (IV) with a nitrous acid.

7. A process for preparing a derivative of nitrosourea represented by the formula (I) as defined in claim 1, which process comprises reacting an amino-alcohol represented by the formula (II) as defined in claim 6 with N-(2-chloroethyl-N-nitrosocarbamylazide or p-nitrophenyl N-(2-chloroethyl)-N-nitrosocarbamate.

8. A pharmaceutical composition comprising as active ingredient a derivative of nitrosourea represented by the formula (I) as defined in claim 1, together with a pharmaceutically acceptable carrier and/or adjuvant.

9. A pharmaceutical composition according to claim 8 in unit dosage form.

10. A compound of the formula (IV) as defined in claim 6.

FIG. 1

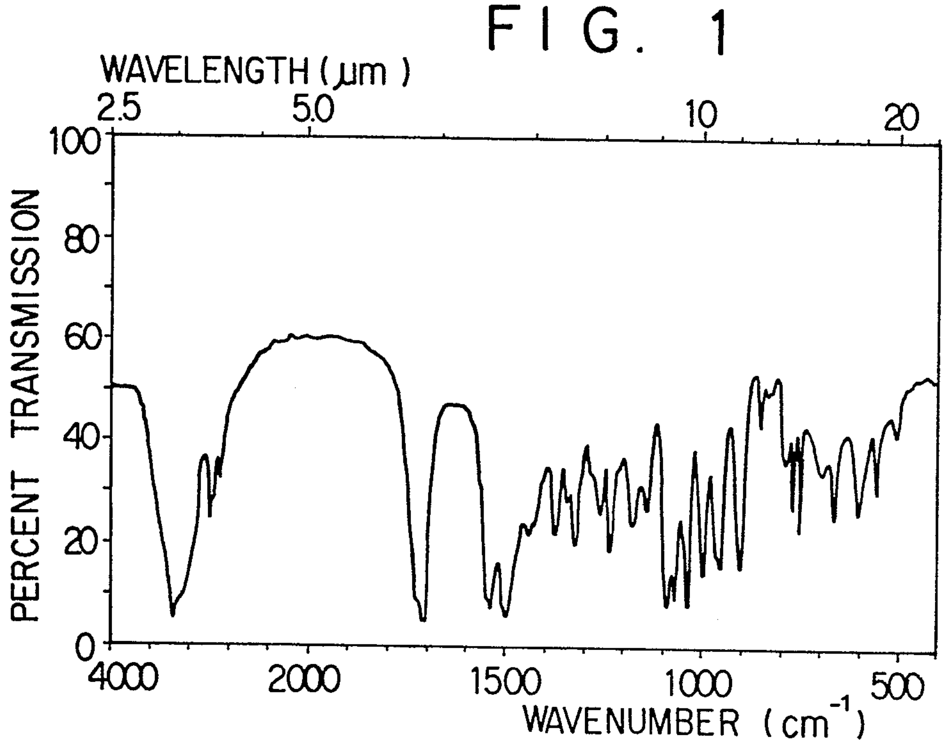
WAVELENGTH (µm)
2.5
5.0
10
20
100
80
60
40
20
0
PERCENT TRANSMISSION
4000
2000
1500
1000
500
WAVENUMBER (cm-1)

FIG. 2

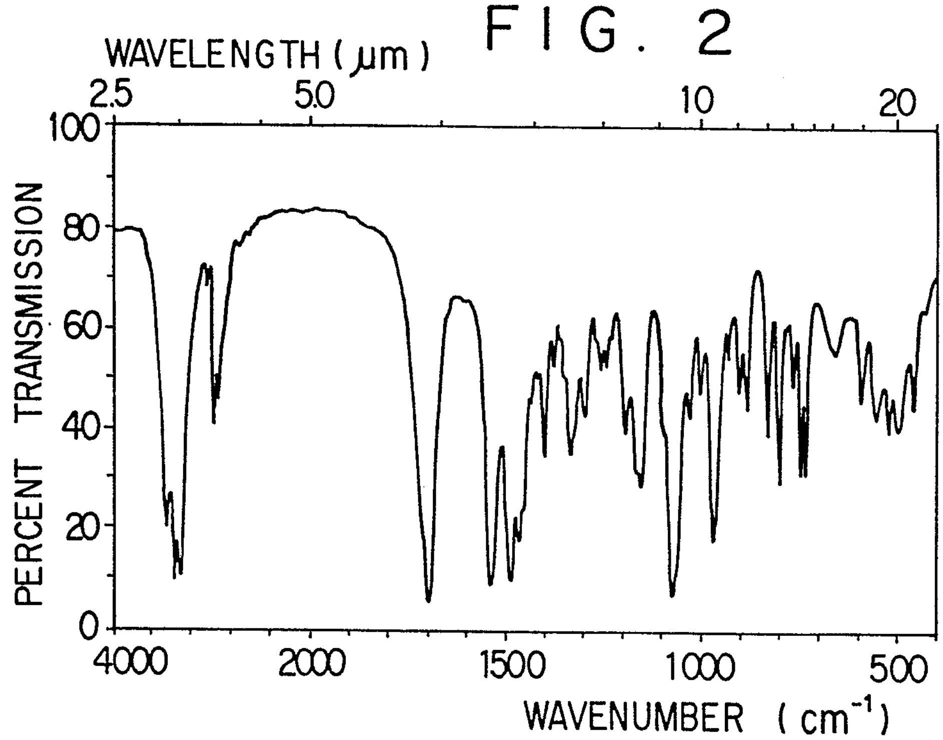
WAVELENGTH (µm)
2.5
5.0
10
20
100
80
60
40
20
0
PERCENT TRANSMISSION
4000
2000
1500
1000
500
WAVENUMBER (cm-1)

F I G. 3

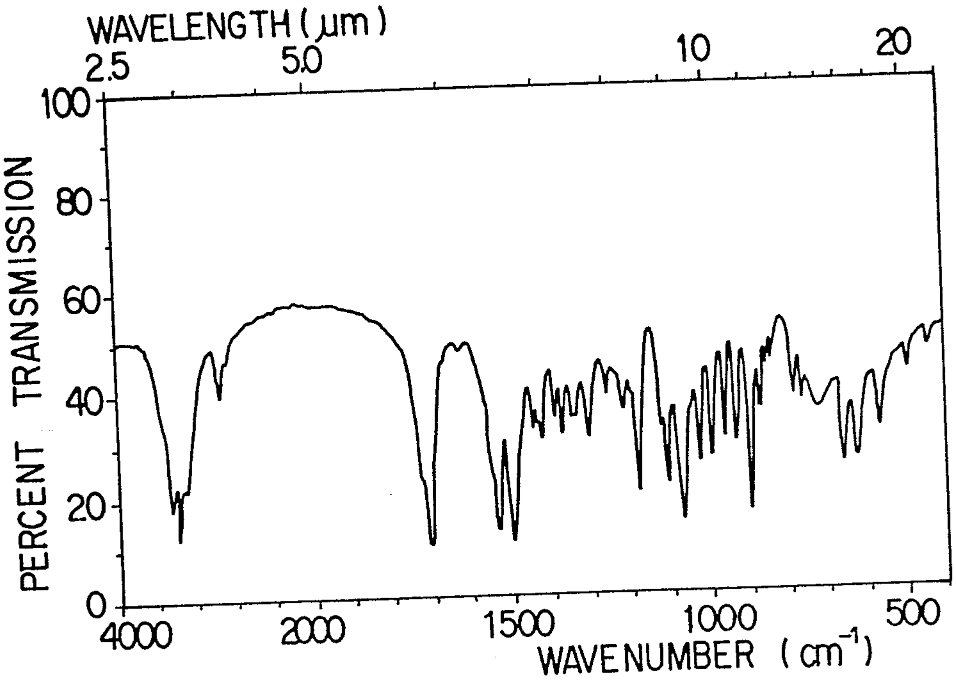
WAVELENGTH (μm)
2.5
5.0
10
20
100
80
60
40
20
0
PERCENT TRANSMISSION
4000
2000
1500
1000
500
WAVENUMBER (cm⁻¹)

F I G. 4

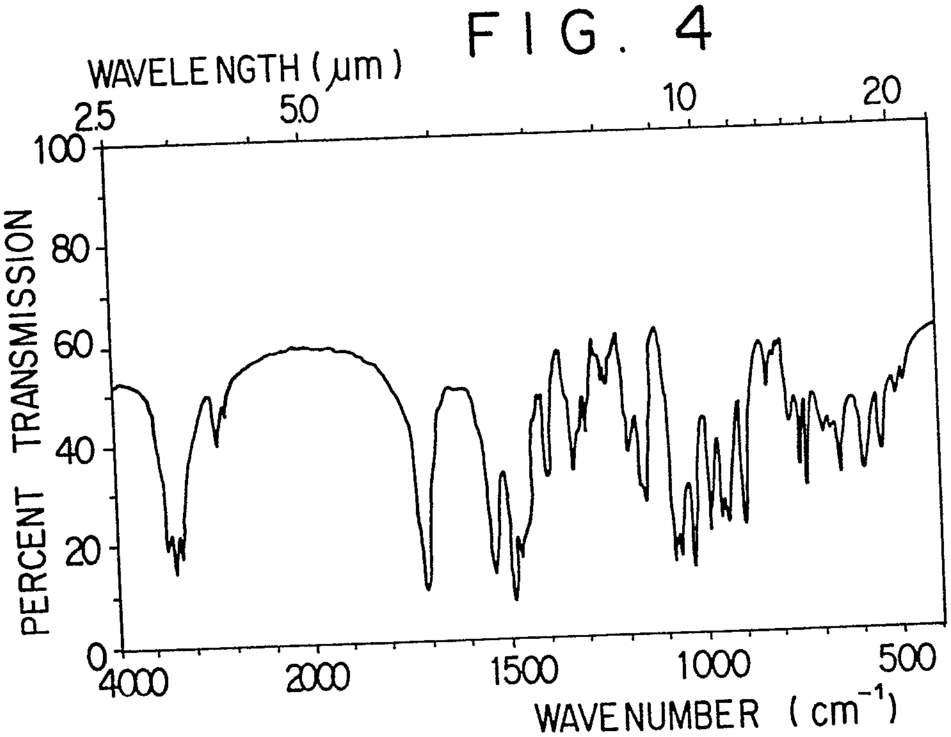
WAVELENGTH (μm)
2.5
5.0
10
20
100
80
60
40
20
0
PERCENT TRANSMISSION
4000
2000
1500
1000
500
WAVENUMBER (cm⁻¹)

FIG. 5

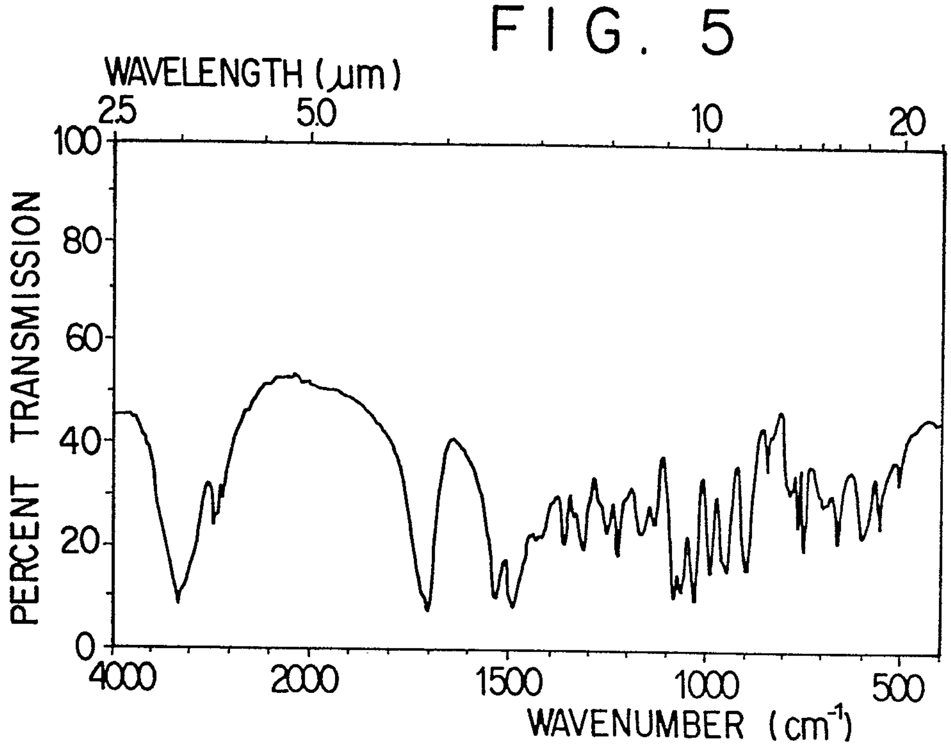
WAVELENGTH (μm)
2.5
5.0
10
20
100
80
60
40
20
0
PERCENT TRANSMISSION
4000
2000
1500
1000
500
WAVENUMBER ($cm^{-1}$)

FIG. 6

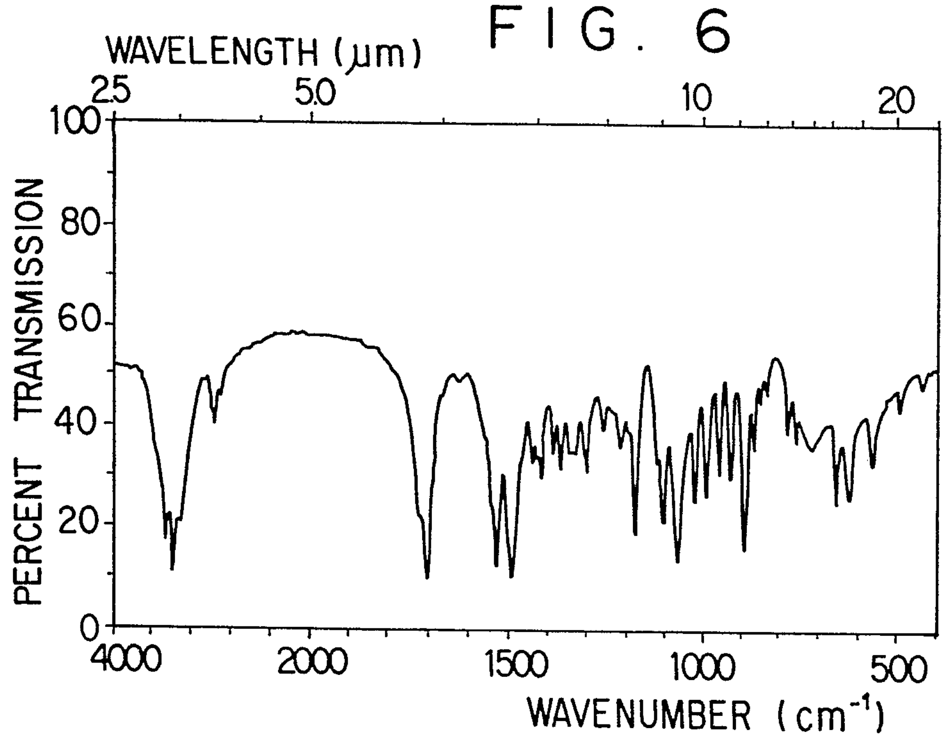
WAVELENGTH (μm)
2.5
5.0
10
20
100
80
60
40
20
0
PERCENT TRANSMISSION
4000
2000
1500
1000
500
WAVENUMBER ($cm^{-1}$)

European Patent Office

# EUROPEAN SEARCH REPORT

0071374
Application number

EP 82 30 3761.9

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| A | US - A - 4 182 757 (TANABE SEIYAKU)<br>* example 62 *<br>-- | 1-10 | C 07 C 127/15<br>A 61 K 31/17 |
| A | Patent Abstracts of Japan<br>Vol. 1, No. 28, 28 March 1977,<br>page 1374C76<br>& JP - A - 51 - 143618<br>---- | 1-10 | |
| | | | TECHNICAL FIELDS SEARCHED (Int.Cl. 3) |
| | | | C 07 C 127/15<br>C 07 C 135/00 |
| | | | CATEGORY OF CITED DOCUMENTS<br>X: particularly relevant if taken alone<br>Y: particularly relevant if combined with another document of the same category<br>A: technological background<br>O: non-written disclosure<br>P: intermediate document<br>T: theory or principle underlying the invention<br>E: earlier patent document, but published on, or after the filing date<br>D: document cited in the application<br>L: document cited for other reasons<br>&: member of the same patent family, corresponding document |
| X | The present search report has been drawn up for all claims | | |

| Place of search | Date of completion of the search | Examiner |
|---|---|---|
| Berlin | 07-10-1982 | BREW |